# NEW EUROPEAN PATENT SPECIFICATION

(11) **EP 1 391 495 B2**
(45) Date of publication and mention of the opposition decision: **21.07.2021**
(45) Mention of the grant of the patent: 19.11.2008
(21) Application number: 02724727.9
(22) Date of filing: 08.05.2002
(51) Int. Cl.: C09K 11/06, H05B 33/14, H05B 33/22, H01L 51/00, H01L 51/50

(54) **ORGANIC ELECTROLUMINESCENCE ELEMENT**
ORGANISCHES ELEKTROLUMINESZENZELEMENT
ELEMENT ELECTROLUMINESCENT ORGANIQUE

(30) Priority: 24.05.2001 JP 2001155290
(43) Date of publication of application: 25.02.2004
(73) Proprietor: IDEMITSU KOSAN CO., LTD., Tokyo 100-8321 (JP)
(72) Inventor: MATSUURA, Masahide, Sodegaura-shi, Chiba 299-0205 (JP); HOSOKAWA, Chishio, Sodegaura-shi, Chiba 299-0205 (JP)
(74) Representative: Hoffmann Eitle
(86) International application number: PCT/JP2002/004485
(87) International publication number: WO 2002/094965

(56) References cited:
- EP-A- 0 666 298
- EP-A1- 0 721 935
- EP-A1- 0 891 121
- EP-A1- 0 915 143
- EP-A2- 0 901 175
- WO-A-01/23344
- WO-A1-01/34584
- JP-A- 9 194 441
- JP-A- 9 301 934
- JP-A- 10 102 052
- JP-A- 11 149 986
- JP-A- 2001 313 178
- US-A- 5 837 166
- BALDO M A ET AL: "VERY HIGH-EFFICIENCY GREEN ORGANIC LIGHT-EMITTING DEVICES BASED ON ELECTROPHOSPHORESCENCE" APPLIED PHYSICS LETTERS, AIP, AMERICAN INSTITUTE OF PHYSICS, MELVILLE, NY, US, vol. 75, no. 1, 5 July 1999 (1999-07-05), pages 4-6, XP000850655 ISSN: 0003-6951
- BALDO M.A. ET AL.: 'Very high-efficiency green organic light-emitting devices based on electrophorescence' APLLIED PHYSICS LETTERS vol. 75, no. 1, 1999, pages 4 - 6, XP000850655
- Adachi, Kwong and Forrest: "Efficient electrophosphorescencence using a doped ambipolar conductive molecular organic thin film", Organic Electronics, vol. 2, 2001, pages 37-43,
- Junji Kido: "Organic electroluminescence materials and displays", 28 February 2001 (2001-02-28), CMC Co., Ltd

## Description

### TECHNICAL FIELD

The present invention relates to an organic electroluminescence device used as a planar light emitting member of wall televisions and a light source for a back light of displays, and more particularly, to an organic electroluminescence device exhibiting a high efficiency of light emission even at a high luminance and a small consumption of electricity.

### BACKGROUND ART

Organic electroluminescence ("electroluminescence" will be referred to as "EL") devices which utilize organic substances are expected to be useful for application as an inexpensive full color display device of the solid light emission type having a great size and various developments on the organic EL devices are being conducted. In general, an organic EL device has a construction comprising a light emitting layer and a pair of electrodes disposed at both sides of the light emitting layer. The light emission of the organic EL device is a phenomenon in which, when an electric field is applied between the two electrodes, electrons are injected from the cathode side and holes are injected from the anode side, the electrons are recombined with the holes in the light emitting layer to form an excited state, and the energy generated when the excited state returns to the ground state is emitted as light.

As the construction of an organic EL device, various constructions have heretofore been known. For examples, in Japanese Patent Application Laid-Open No. Showa 63(1988)-295695, it is disclosed that an aromatic tertiary amine is used as a material for a hole transporting layer in an organic EL device having a construction of ITO (indium tin oxide) / a hole transporting layer / a light emitting layer / a cathode. A luminance as high as several hundred cd/m² can be achieved at an applied voltage of 20 V or lower due to this construction of the device. It is also reported that the efficiency of light emission is about 40 lumens/W or greater at a luminance of several hundred cd/m² or lower when an iridium complex compound which is a dopant for phosphorescent light emission is used as the dopant in the light emitting layer (T. Tsutsui et al., Jpn. J. Appl. Phys., Vol. 38 (1999), pp. L1502 to L1504).

When an organic EL device is applied to a flat panel display, it is required that the efficiency of light emission be improved and the consumption of electricity be decreased. The device having the above construction has a drawback in that a marked decrease in the efficiency of light emission accompanies an improvement in the luminance of the emitted light and a problem arises in that the consumption of electricity of the flat panel display does not decrease due to this drawback. In particular, in the case of the passive driving, a luminance of several thousand cd/m² or greater is required instantaneously for practical applications and the increase in the efficiency of light emission in the high luminance region is important. However, the decrease in the efficiency of light emission cannot be improved as long as the hole transporting materials currently used for practical applications is used since the deactivation process of the triplet state is dominant in the high luminance region.

M. Baldo et. al., Applied Physics Letters, Vol. 75 (1999), 4-6 discloses an organic electroluminescent device with an organometallic complex in the light-emitting layer and an amine derivative as a hole transporting material. EP-A1-721935 discloses amine derivatives for the hole-transporting layer of organic electroluminescent devices. The light emitting layer contains fluorescent light emitting materials.

### DISCLOSURE OF THE INVENTION

The present invention has been made to overcome the above problem and has an object of providing an organic EL device exhibiting a high efficiency of light emission even at a high luminance of several thousand cd/m² or greater and a small consumption of electricity.

As the result of intensive studies by the present inventors to develop a material for an organic EL device having the above desirable properties, it was found that the object could be achieved by utilizing amine derivatives represented by general formula (III) shown in the following and preferably especially arylamine derivatives or having no polyacene-based condensed aromatic structures. The present invention has been completed based on this knowledge.

The present invention provides an organic EL device which comprises a pair of electrodes and an organic medium which comprises a hole transporting layer comprising a hole transporting material and a light emitting layer plurality of layers comprising an organometallic complex compound having a heavy metal the light emitting layer and is disposed between the pair of electrodes,
wherein said light emitting material is a phosphorescent light emitting material and wherein the heavy metal is selected from Ir, Pt, Pd, Ru, Rh, Mo and Re, the hole transporting material comprises an arylamine derivative having no polyacene-based condensed aromatic structures, and the arylamine derivative is represented by following general formula (III): 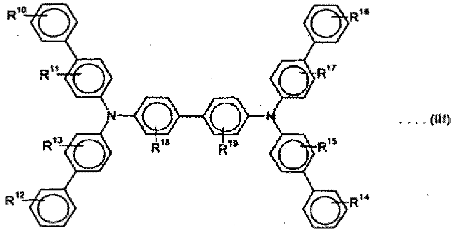 wherein R¹⁰ to R¹⁹ each independently represent hydrogen atom, an alkyl group having 1 to 6 carbon atoms, an alkoxyl group having 1 to 6 carbon atoms or a phenyl group, and the atoms or the groups represented by R¹⁰ to R¹⁹ may be a same with or different from each other.

Examples the construction of the organic EL device of the present invention include (1) an anode / an organic light emitting layer / a cathode, (2) an anode / a hole transporting layer / an organic light emitting layer / a cathode, (3) an anode / an organic light emitting layer / an electron injecting layer / a cathode and (4) an anode / a hole transporting layer / an organic light emitting layer / an electron injecting layer / a cathode. The organic EL device of the present invention may have any of the above constructions (1) to (4) as long as the amine derivative represented by general formula (III) is comprised in the hole transporting layer in the organic medium disposed between the pair of electrodes (the anode and the cathode). The organic medium means the organic light emitting layer in construction (1), the combination of the hole transporting layer and the organic light emitting layer in construction (2), the combination of the organic light emitting layer and the electron injecting layer in construction (3) and the combination of the hole transporting layer, the organic light emitting layer and the electron injecting layer in construction (4).

The hole transporting layer may be a layer having a single layer structure composed of the amine derivative alone, a layer having a multi-layer structure comprising a layer comprising the amine derivative and layers comprising conventional hole transporting materials, or a layer which has a single layer structure or a multi-layer structure and comprises a mixture of the amine derivative and conventional hole transporting materials.

The hole transporting layer comprising the amine derivative can be formed using the amine derivative and, where necessary, other hole transporting materials in accordance with the vacuum vapor deposition process, the casting process, the coating process or the spin coating process. The hole transporting layer can also be formed in accordance with the casting process, the coating process or the spin coating process using a fluid containing the amine derivative dispersed in a transparent polymer such as polycarbonate, polyurethane, polystyrene, polyarylate and polyester, or in accordance with the simultaneous vapor deposition of the amine derivative and the transparent polymer.

The organic light emitting layer may be a layer having a single layer structure composed of the amine derivative alone, a layer having a multi-layer structure comprising a layer comprising the amine derivative and layers comprising conventional organic light emitting materials, or a layer which has a single layer structure or a multi-layer structure and comprises a mixture of the amine derivative and conventional light emitting materials.

The organic light emitting layer comprising the amine derivative can be formed using the amine derivative and, where necessary, other organic light emitting materials in accordance with the vacuum vapor deposition process, the casting process, the coating process or the spin coating process.

As the materials for preparing the organic EL device of the present invention other than the amine derivative and the organometallic complex compound having a heavy metal, materials conventionally used for preparation of organic EL devices can be used.

The organometallic complex compound having a heavy metal is not particularly limited. It is preferable that the organometallic complex compound works as the dopant for light emission. Examples of the heavy metal include Ir, Pt, Pd, Ru, Rh, Mo and Re. As the ligand in the organometallic complex compound, ligands bonded to or coordinating the metal through C and N (CN ligands) can be used. Examples of such ligand include the following ligands: 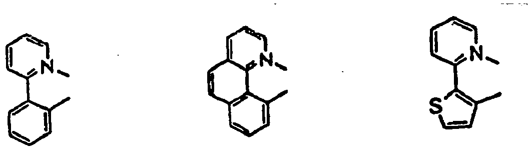 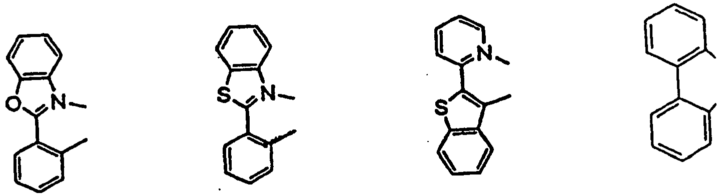 and derivatives of these ligands. Examples of the substituent include alkyl groups, alkoxyl group, phenyl group, polyphenyl group and naphthyl group.

As the organometallic complex compound, compounds having 2 to 4 ligands shown above coordinating the heavy metal are preferable. The CN ligands are preferable as described above and organometallic complex compounds having mixed ligands comprising the CN ligands and ligands bonded to or coordinating the metal through O such as diketone derivatives, alkyl-O- and aryl-O- are also preferable.

It is preferable that the organometallic complex compound has the light emitting property to which the triplet state contributes and more preferably the light emitting property to which the triplet state contributes at the room temperature. In the light emission to which the triplet state contributes, the deactivation process of the excited state of the triplet is suppressed since the content of the polyacene-based condensed ring structure such as naphthalene and anthracene in the amine derivative is small. Therefore, the light emission to which the triplet state contributes can be efficiently performed and the device exhibiting a small consumption of electricity can be obtained.

The light emitting material comprising the organometallic complex compound is a phosphorescent light emitting material.

In the organic EL device of the present invention, the main material of the organic light emitting layer is not particularly limited and conventional materials can be used. Examples of such materials include the above amine derivatives, carbazole derivatives, oxadiazole derivatives, triazole derivatives, fluorescent brighteners such as benzoxazole-based fluorescent brighteners, benzothiazole-based fluorescent brighteners and benzimidazole-based fluorescent brighteners, oxanoid compounds chelating metals and distyrylbenzene-based compounds, which have the excellent property for forming a thin film.

It is preferable that the main material of the organic light emitting layer has an energy of the triplet level greater than an energy of the triplet level of the organometallic complex compound having a heavy metal. When this condition is satisfied, the energy of the main material of the organic light emitting layer is transferred efficiently to the organometallic complex compound and the efficiency of light emission is further increased.

In the organic EL device of the present invention, the material for the electron injecting layer is not particularly limited and conventional materials can be used. Examples of such materials include organometallic complex compounds such as tris(8-quinolinolato)aluminum, tris(8-quinolinolato)gallium and bis(10-benzo[h]quinolinolato)beryllium, oxadiazole derivatives, triazole derivatives, triazine derivatives, perylene derivatives, quinoline derivatives, quinoxaline derivatives, diphenylquinone derivatives, fluorenone derivatives substituted with nitro group and thiopyrane dioxide derivatives. The electron injecting layer may have a single layer structure or a multi-layer structure. The electron injecting layer may have a hole barrier layer having the hole barrier property, which is the property exhibiting the function of enclosing electrons within the light emitting layer, i.e., the property having an ionization potential greater than the ionization potential of the material used for the light emitting layer. Examples of the specific compound having the hole barrier property include phenanthroline derivatives. A still higher efficiency of light emission can be obtained when the electron injecting property is enhanced by adding an alkali metal, an alkaline earth metal, a rare earth metal, an alkali compound, an alkaline earth compound, a rare earth compound or an alkali metal coordinated with an organic compound to the electron injecting layer.

In the organic EL device of the present invention, inorganic materials may be added to the hole transporting layer and the electron injecting layer, where necessary.

It is preferable that the organic EL device of the present invention is supported with a substrate. The material for the substrate is not particularly limited and materials conventionally used for organic EL devices such as glasses, transparent plastics and quartz can be used.

As the material for the anode, metals, alloys, electrically conductive compounds and mixtures of these materials, which have a work function of 4 eV or greater, are preferable. Examples of the material for the anode include metals such as Au and dielectric transparent materials such as CuI, ITO, SnO₂ and ZnO. The anode can be prepared by forming a thin film of the electrode material described above in accordance with a process such as the vapor deposition process and the sputtering process. When the light emitted from the organic light emitting layer is obtained through the anode, it is preferable that the anode has a transmittance of the emitted light greater than 10%. It is also preferable that the sheet resistivity of the anode is several hundred Ω/□ or smaller. The thickness of the anode is, in general, selected in the range of 10 nm to 1 µm and preferably in the range of 10 to 200 nm although the preferable range may be different depending on the used material.

As the material for the cathode, metals, alloys, electrically conductive compounds and mixtures of these materials, which have a work function of 4 eV or smaller, are preferable. Examples of the material for the anode include sodium, lithium, aluminum, mixtures of magnesium and silver, mixtures of magnesium and copper, Al/Al₂O₃ and indium.

The cathode can be prepared by forming a thin film of the electrode material described above in accordance with a process such as the vapor deposition process and the sputtering process. When the light emitted from the organic light emitting medium layer is obtained through the cathode, it is preferable that the cathode has a transmittance of the emitted light greater than 10 %. It is also preferable that the sheet resistivity of the cathode is several hundred Ω/□ or smaller. The thickness of the cathode is, in general, selected in the range of 10 nm to 1 µm and preferably in the range of 50 to 200 nm although the preferable range may be different depending on the material used.

It is preferable that at least one of the anode and the cathode is formed with a transparent or translucent material so that the light emitted from the organic light emitting layer is efficiently obtained at the outside.

The present invention will be described more specifically with reference to examples in the following.

### Example 1 (Reference)

A glass substrate (manufactured by GEOMATEC Company) of 25 mm × 75 mm × 1.1 mm thickness having an ITO transparent electrode was cleaned by application of ultrasonic wave in isopropyl alcohol for 5 minutes and then by exposure to ozone generated by ultraviolet light for 30 minutes. The glass substrate having the transparent electrode lines which had been cleaned was attached to a substrate holder of a vacuum vapor deposition apparatus and cleaned with plasma under the atmosphere of a mixture of oxygen and argon. On the surface of the cleaned substrate at the side having the transparent electrode lines, a film of compound 1 shown below, having a thickness of 50 nm was formed in a manner such that the formed film covered the transparent electrode. The formed film of compound 1 worked as the hole transporting layer. On the formed film, 4,4'-N,N'-dicarbazolebiphenyl (CBP) and tris(2-phenylpyridyl)iridium (Ir(Ppy)) were binary vapor deposited in a manner such that the content of Ir(Ppy) in the light emitting layer composed of CBP and Ir(Ppy) was controlled at 8% by weight and a film was formed. The formed film worked as the light emitting layer. On the formed light emitting layer, a film of 2,9-dimethyl-4,7-diphenyl-1,10-phenanthrone (BCP) having a thickness of 10 nm and a film of tris(8-quinolinol)aluminum (an Alq film) having a thickness of 40 nm were laminated. The BCP film worked as the hole barrier layer and the Alq film worked as the electron injecting layer. Thereafter, Li (the source of lithium: manufactured by SAES GETTERS Company) of the alkali metal and Alq were binary vapor deposited and an Alq:Li film was formed as the electron injecting layer (the cathode). On the formed Alq:Li film, metallic aluminum was vapor deposited to form a metal cathode and an organic EL device was prepared.

The luminance of the obtained organic EL device was measured under various voltages and electric currents and the efficiency of light emission (=(luminance)/(current density)) at the luminance of emitted light of 10,000 cd/m² was calculated. The efficiency of light emission was found to be 35 cd/A.

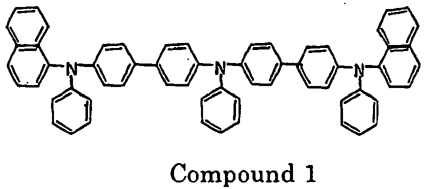

### Example 2 (Reference)

An organic EL device was prepared in accordance with the same procedures as those conducted in Example 1 except that compound 2 shown in the following was used in place of compound 1.

The luminance of the obtained organic EL device was measured under various voltages and electric currents and the efficiency of light emission (=(luminance)/(current density)) at the luminance of emitted light of 10,000 cd/m² was calculated. The efficiency of light emission was found to be 37 cd/A.

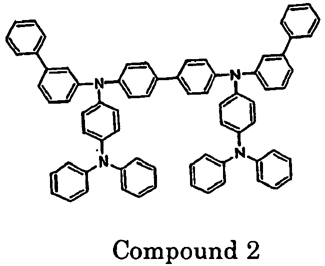

### Example 3

An organic EL device was prepared in accordance with the same procedures as those conducted in Example 1 except that compound 3 shown in the following was used in place of compound 1.

The luminance of the obtained organic EL device was measured under various voltages and electric currents and the efficiency of light emission (=(luminance)/(current density)) at the luminance of emitted light of 10,000 cd/m² was calculated. The efficiency of light emission was found to be 50 cd/A.

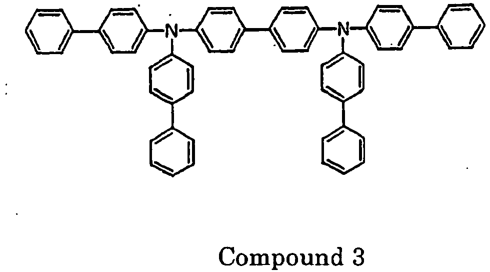

### Example 4 (Reference)

An organic EL device was prepared in accordance with the same procedures as those conducted in Example 1 except that compound 4 shown in the following was used in place of compound 1.

The luminance of the obtained organic EL device was measured under various voltages and electric currents and the efficiency of light emission (=(luminance)/(current density)) at the luminance of emitted light of 10,000 cd/m² was calculated. The efficiency of light emission was found to be 40 cd/A.

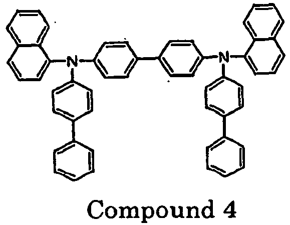

### Comparative Example 1

An organic EL device was prepared in accordance with the same procedures as those conducted in Example 1 except that compound 5 shown in the following was used in place of compound 1.

The luminance of the obtained organic EL device was measured under various voltages and electric currents and the efficiency of light emission (=(luminance)/(current density)) at the luminance of emitted light of 10,000 cd/m² was calculated. The efficiency of light emission was found to be 25 cd/A.

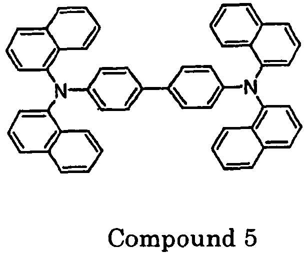

### Example 5 (Reference)

An organic EL device was prepared in accordance with the same procedures as those conducted in Example 1 except that compound 6 shown in the following was used in place of compound 1.

The luminance of the obtained organic EL device was measured under various voltages and electric currents and the efficiency of light emission (=(luminance)/(current density)) at the luminance of emitted light of 10,000 cd/m² was calculated. The efficiency of light emission was found to be 40 cd/A.

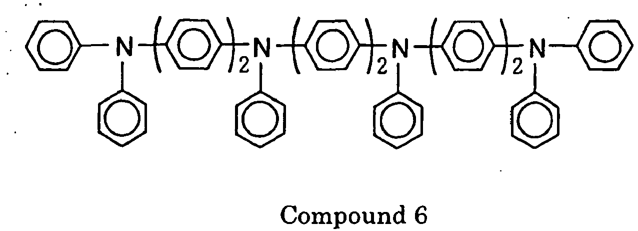

### Comparative Example 2

An organic EL device was prepared in accordance with the same procedures as those conducted in Example 1 except that NPD shown in the following was used in place of compound 1.

The luminance of the obtained organic EL device was measured under various voltages and electric currents and the efficiency of light emission (=(luminance)/(current density)) at the luminance of emitted light of 10,000 cd/m² was calculated. The efficiency of light emission was found to be 20 cd/A, which was inferior to those in Examples 1 to 5.

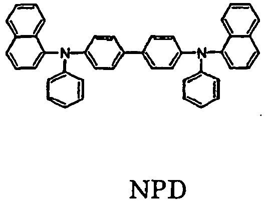

In Example 3 and Reference Examples 2 and 5, the efficiency of light emission was remarkably high since the aryl amine derivative or polyarylamine derivative having no polyacene-based condensed aromatic structures was used.

### INDUSTRIAL APPLICABILITY

As described in detail in the above, the organic EL device of the present invention exhibits a high efficiency of light emission at luminances as high as several thousand cd/m² and a small consumption of electricity and can be advantageously used as a device used at a high luminance such as a flat panel display.

## Claims

1. An organic electroluminescence device which comprises a pair of electrodes and an organic medium which comprises at least
a hole transporting layer comprising a hole transporting material and
a light emitting layer comprising a light emitting material comprising an organometallic complex compound having a heavy metal and is disposed between the pair of electrodes,
wherein
said light emitting material is a phosphorescent light emitting material and wherein the heavy metal is selected from Ir, Pt, Pd, Ru, Rh, Mo and Re,
the hole transporting material comprises an arylamine derivative having no polyacene-based condensed aromatic structures, and
the arylamine derivative is represented by following general formula (III): 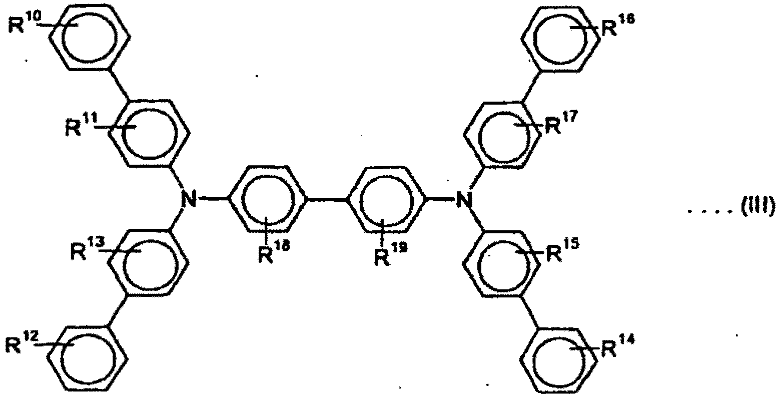 wherein R¹⁰ to R¹⁹ each independently represent hydrogen atom, an alkyl group having 1 to 6 carbon atoms, an alkoxyl group having 1 to 6 carbon atoms or a phenyl group, and
the atoms or the groups represented by R¹⁰ to R¹⁹ may be a same with or different from each other.

2. The organic electroluminescence device according to Claim 1, wherein the main material constituting the light emitting material has an energy of a triplet level greater than the energy of a triplet level of the organometallic complex compound having a heavy metal.

3. The organic electroluminescence device according to Claim 1 or 2, wherein a ligand in the organometallic complex compound having a heavy metal comprises at least one ligand selected from following ligands: 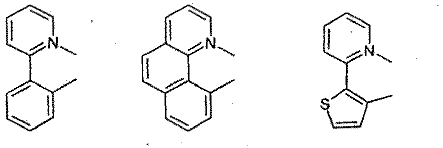 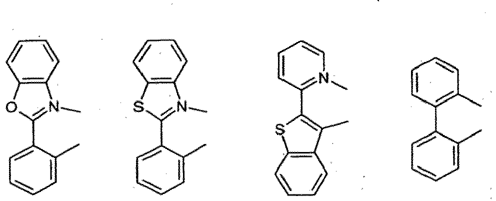 and derivatives thereof.

4. The organic electroluminescence device according to Claim 3, wherein the organometallic complex compound has 2 to 4 said ligands coordinating the heavy metal.

## Patentansprüche

1. Organische Elektrolumineszenzvorrichtung, die ein Paar von Elektroden und ein organisches Medium enthält, die zumindest enthält:
eine Lochtransportschicht, enthaltend ein Lochtransportmaterial, und
eine lichtemittierende Schicht, die ein lichtemittierendes Material enthält, das eine organometallische Komplexverbindung mit einem Schwermetall enthält, und die zwischen dem Paar von Elektroden angeordnet ist, worin
das lichtemittierende Material ein phosphoreszierendes lichtemittierendes Material ist und worin das Schwermetall ausgewählt ist aus Ir, Pt, Pd, Ru, Rh, Mo und Re,
das Lochtransportmaterial ein Arylaminderivat ohne Polyacen-basierte kondensierte aromatische Strukturen enthält und
das Arylaminderivat durch folgende allgemeine Formel (III) dargestellt ist: 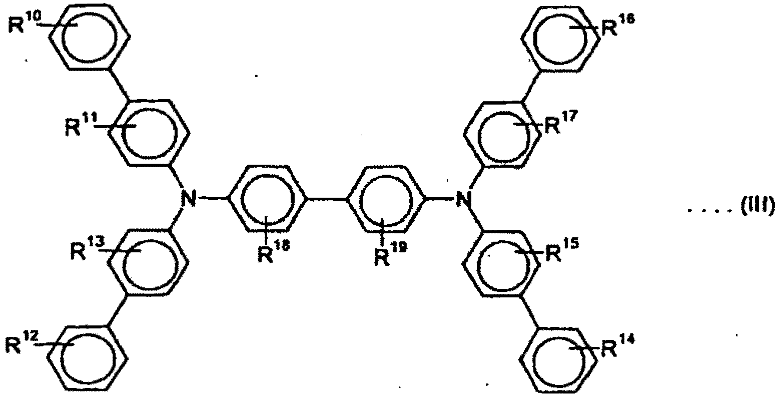 worin R¹⁰ bis R¹⁹ jeweils unabhängig ein Wasserstoffatom, eine Alkylgruppe mit 1 bis 6 Kohlenstoffatomen, Alkoxylgruppe mit 1 bis 6 Kohlenstoffatomen oder Phenylgruppe sind und
die Atome oder Gruppen, dargestellt durch R¹⁰ bis R¹⁹, gleich oder verschieden voneinander sein können.

2. Organische Elektrolumineszenzvorrichtung gemäß Anspruch 1, worin das Hauptmaterial, das das lichtemittierende Material konstituiert, eine Energie eines Tripletniveaus aufweist, die größer ist als die Energie eines Tripletniveaus der organometallischen Komplexverbindung mit einem Schwermetall.

3. Organische Elektrolumineszenzvorrichtung gemäß Anspruch 1 oder 2, worin ein Ligand in der organometallischen Komplexverbindung mit einem Schwermetall zumindest einen Liganden enthält, ausgewählt aus folgenden Liganden: 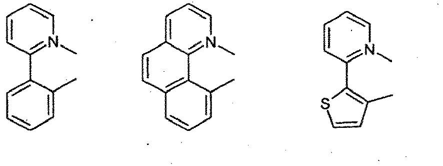 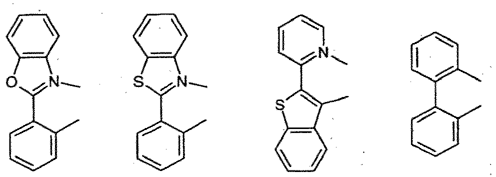 und Derivaten davon.

4. Organische Elektrolumineszenzvorrichtung gemäß Anspruch 3, worin die organometallische Komplexverbindung 2 bis 4 der Liganden aufweist, die das Schwermetall koordinieren.

## Revendications

1. Dispositif à électroluminescence organique qui comprend une paire d'électrodes et un milieu organique qui comprend au moins
une couche de transport de trous comprenant un matériau transportant les trous et
une couche émettant de la lumière comprenant un matériau émettant de la lumière comprenant un composé complexe organométallique contenant un métal lourd et est disposée entre la paire d'électrodes,
dans lequel
ledit matériau émettant de la lumière est un matériau émettant de la lumière par phosphorescence et dans lequel le métal lourd est choisi parmi Ir, Pt, Pd, Ru, Rh, Mo et Re,
le matériau transportant les trous comprend un dérivé arylamine ne comportant pas de structures aromatiques condensées dérivées de polyacène, et
le dérivé arylamine est représenté par la formule générale (III) suivante : 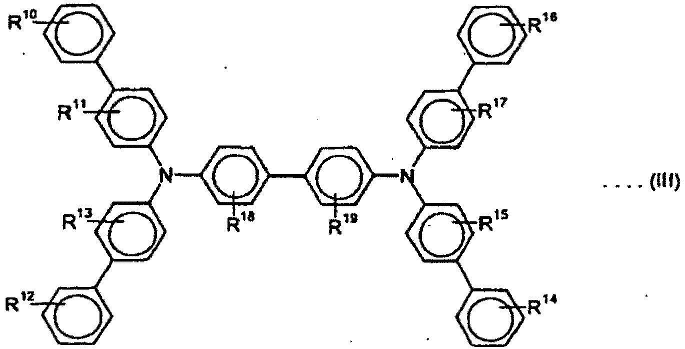 dans laquelle R¹⁰ à R¹⁹ représentent indépendamment les uns des autres un atome d'hydrogène, un groupe alkyle comportant de 1 à 6 atomes de carbone, un groupe alcoxyle comportant de 1 à 6 atomes de carbone ou un groupe phényle, et
les atomes ou les groupes représentés par R¹⁰ à R¹⁹ peuvent être identiques ou différents les uns des autres.

2. Le dispositif à électroluminescence organique selon la revendication 1, dans lequel le matériau principal constituant le matériau émettant de la lumière a une énergie d'un niveau de triplet supérieure à l'énergie d'un niveau de triplet du composé complexe organométallique contenant un métal lourd.

3. Le dispositif à électroluminescence organique selon la revendication 1 ou 2, dans lequel un ligand dans le composé complexe organométallique contenant un métal lourd comprend au moins un ligand choisi parmi les ligands suivants : 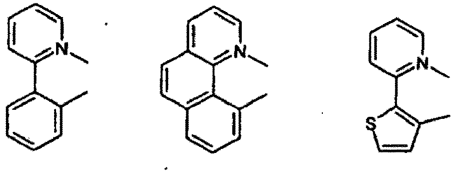 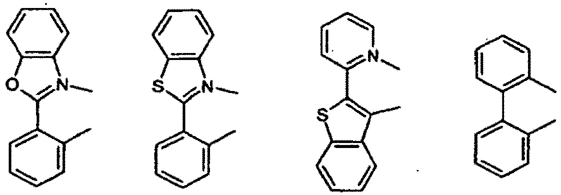 et leurs dérivés.

4. Le dispositif à électroluminescence organique selon la revendication 3, dans lequel le composé complexe organométallique comprend de 2 à 4 desdits ligands coordonnant le métal lourd.
